# EUROPEAN PATENT APPLICATION

(11) **EP 3 006 560 A1**
(43) Date of publication of application: **13.04.2016**
(21) Application number: 14188341.3
(22) Date of filing: 09.10.2014
(51) Int. Cl.: C12N 5/076

(54) **Miwi2 as a marker for spermatogonial stem cells and method for diagnosing spermatogonial stem cells based on said marker**

(71) Applicant: European Molecular Biology Laboratory, 69117 Heidelberg (DE)
(72) Inventor: O'Carroll, Dónal, 69117 Heidelberg (DE); Carrieri, Claudia, 00015 Monterotondo (Rome) (IT)
(74) Representative: Krauss, Jan

(57) **Abstract**

The present invention relates to isolated mammalian spermatogonial stem cells (SSCs) that are Miwi2/Piwil4^{Hi} c-kit^{Neg}, pharmaceutical compositions, and *in vitro* methods for detecting a mammalian spermatogonial stem cell population. The methods can be performed in the context of testicular reconstitution activity, such as self-renewal or contribution to testicular homeostasis and regeneration or testicular cancer.

## Description

The present invention relates to isolated mammalian spermatogonial stem cells (SSCs) that are Miwi2/Piwi14^{Hi} c-kit^{Neg}, pharmaceutical compositions, and *in vitro* methods for detecting a mammalian spermatogonial stem cell population. The methods can be performed in the context of testicular reconstitution activity, such as self-renewal or contribution to testicular homeostasis and regeneration or testicular cancer.

### Background of the invention

The spermatogonial stem cell (SSC) supports spermatogenesis throughout adult life. This stem cell population resides within the A type undifferentiated spermatogonia, with GFRα1-expressing cells recognized as the population that maintains testicular homeostasis. The SSC populations that underpin the regenerative capacity of the testis are not fully resolved.

Spermatogonial stem cells have been narrowed down to single (Aₛ) and aligned (Aₐ₁) type A undifferentiated spermatogonia. Spermatogonia upon division fail to complete cytokinesis and remain connected with inter-cytoplasmic bridges. Thus, the original Aₛ model proposed that Aₛ represented the SSC, given that they reside at the apex of undifferentiated spermatogonia.

Carmell et al. (in: MIWI2 is essential for spermatogenesis and repression of transposons in the mouse male germline. Dev Cell. 2007 Apr;12(4):503-14. Epub 2007 Mar 29) discloses that small RNAs associate with Argonaute proteins and serve as sequence-specific guides for regulation of mRNA stability, productive translation, chromatin organization, and genome structure. In animals, the Argonaute superfamily segregates into two clades. The Argonaute clade acts in RNAi and in microRNA-mediated gene regulation in partnership with 21-22 nt RNAs. The Piwi clade, and their 26-30 nt piRNA partners, with important functions in transposon silencing in the germ line. In mice, the 3 Piwi-family members have been demonstrated to have essential roles in spermatogenesis. Here, the inventors examine the effects of disrupting the gene encoding the third family member, MIWI2. Miwi2-deficient mice display a meiotic-progression defect in early prophase of meiosis I and a marked and progressive loss of germ cells with age. These phenotypes may be linked to an inappropriate activation of transposable elements detected in Miwi2 mutants. These observations suggest a conserved function for Piwi-clade proteins in the control of transposons in the germline.

Deng et al. (in: miwi, a murine homolog of piwi, encodes a cytoplasmic protein essential for spermatogenesis. Dev Cell. 2002 Jun;2(6):819-30) describe that the Piwi family genes are crucial for stem cell self-renewal, RNA silencing, and translational regulation in diverse organisms. They report the cloning of a murine Piwi gene Miwi (Piwil1) essential for spermatogenesis. Miwi encodes a cytoplasmic protein specifically expressed in spermatocytes and spermatids. Miwi(null) mice display spermatogenic arrest at the beginning of the round spermatid stage, resembling the phenotype of CREM, a master regulator of spermiogenesis. Furthermore, mRNAs of ACT (activator of CREM in testis) and CREM target genes are downregulated in miwi(null) testes. Whereas MIWI and CREM do not regulate each other's expression, MIWI complexes with mRNAs of ACT and CREM target genes. Hence, MIWI may control spermiogenesis by regulating the stability of these mRNAs. Miwi has been definitely shown to post-transcriptionally cleave expressed LINE1 elements during spermiogenesis.

Gu et al. (in: Genetic variants in Piwi-interacting RNA pathway genes confer susceptibility to spermatogenic failure in a Chinese population. Hum Reprod. 2010 Dec;25(12):2955-61. doi: 10.1093/humrep/deq274. Epub 2010 Oct 11) describes that the Piwi subfamily of genes is involved in spermatogenesis for the maintenance and meiosis of germ line stem cells. Mice bearing targeted mutations in Piwi genes (Miwi, Mili (Piwil2) and Miwi2) are sterile with distinct defects in spermatogenesis. They hypothesized that Piwi gene polymorphisms could be a risk factor for spermatogenic failure, and present the first epidemiologic evidence supporting the involvement of genetic polymorphisms in Piwi genes in spermatogenic failure.

Singh et al. (in: Spermatogonial stem cells, infertility, and testicular cancer, J Cell Mol Med. 2011 March ; 15(3): 468-483) describe that SSCs not only maintain normal spermatogenesis, but also sustain fertility by critically balancing both SSC self-renewal and differentiation. This self-renewal and differentiation in turn is tightly regulated by a combination of intrinsic gene expression within the SSC as well as the extrinsic gene signals from the niche. Increased SSCs self-renewal at the expense of differentiation result in germ cell tumors, on the other hand, higher differentiation at the expense of self-renewal can result in male sterility. Testicular germ cell cancers are the most frequent cancers among young men in industrialized countries. They believe that studying the biology of SSCs will not only provide better understanding of stem cell regulation in the testis but eventually will also be a novel target for male infertility and testicular cancers.

Recent data have also shown that the cell surface receptor GFRα1 identifies a sub-population of Aₛ, A_{paired} (Aₚᵣ) and Aₐₗ₄ cells that support long-term spermatogenic homeostasis in mice. Furthermore, this population was also shown to be highly dynamic with fragmentation of GFRα1-positive (GFRα1^{Pos}) Aₚᵣ and Aₐₗ₄, replenishing the pool of Aₛ spermatogonia. After the Aₐₗ₈₋₁₆ stage, cells upregulate the surface receptor c-Kit and become differentiating spermatogonia. These differentiating spermatogonia can be considered as transit amplifying cells committed to terminal differentiation. In fact from Aₛ stage to meiotic entry, there is a massive amplification of spermatogonia with a synchronous 10-11 cell divisions over approximately 11 days. An interesting facet of Aₛ and Aₐₗ is the heterogeneity in gene expression observed within these populations in terms of both transcription factors (Plzf, ID4, Ngn3), surface receptor (GFRα1) as well as RNA binding proteins (Nanos2 and 3).

This heterogeneity hints at the presence of distinct Aₛ/Aₐₗ populations with likely differential potency with regard to self-renewal or contribution to testicular homeostasis and regeneration.

Thus, the inventors sought to identify novel undifferentiated spermatogonial populations and characterize their contribution to testicular physiology and disease. Thus, in a first aspect of the present invention, this object of the present invention is solved by providing an isolated mammalian spermatogonial stem cell (SSC) that is Miwi2/Piwi14^{Hi} c-kit^{Neg}. Preferred is the SSC according to the present invention which is further selected from GFRα1^{Neg}, low side scatter (SSC^{lo}), and CD9, CD49f, Thy-1, CD29, and CD24 positivity.

Through the analysis of Miwi2^{Tom} transcriptional reporter allele, the inventors found that Miwi2 expression defines a tiny population of c-Kit negative predominantly quiescent cells that express all the surface markers as well as other genes that have been associated with SSC activity. This population comprises a fraction of the primitive A type undifferentiated spermatogonia with a Miwi2-Tom^{Hi} sub-population being GFRα1 negative. This Miwi2-Tom^{Hi} c-kit^{Neg} GFRα1^{Neg} population confers robust reconstitution activity in transplantation assays. To resolve the contribution Miwi2-expressing A type spermatogonia population to the maintenance of the testis the inventors performed a lineage depletion experiment. The loss of Miwi2-expressing cells had a transient impact on testicular homeostasis but was essential for the effective regenerative capacity of the testis after injury. In summary, the present invention provides a Miwi2-expressing SSC population that is essential for efficient testicular regeneration, and the maintenance of testicular homeostasis and its regenerative capacity.

The Piwil4 gene is conserved in human (database accession NM_152431.2), chimpanzee, dog, cow, rat, and frog. Thus, a mammal in the context of the present invention is selected from a human, chimpanzee, dog, cow, sheep, cat, mouse, rat, and frog.

Another aspect of the present invention then relates to a pharmaceutical composition, comprising an SSC according to the present invention, and a pharmaceutically acceptable carrier. Pharmaceutical compositions comprising stem cells are known in the art, in particular for stem cell transplantation. Commonly used carriers are suitable buffers that further contain compounds that suitable to promote viability and freshness of the stem cells, such as, for example DMSO.

Excluded from the present invention are human totipotent embryonic stem cells.

Another aspect of the present invention then relates to an *in vitro* method for detecting a mammalian spermatogonial stem cell population, comprising detecting the expression of the stem cell markers Miwi2/Piwil4 and c-kit, optionally together with the markers GFRα1, SSC^{lo}, CD9, CD49f, Thy-1, CD29, and CD24. Common methods for detecting markers, such as protein markers, in stem cells include FACS, immunohistochemistry, and western blots, the use of antibodies, as well as immunohistochemical analyses. Additional methods involve rtPCR and/or mRNA expression analyses.

Preferred is an *in vitro* method for detecting testicular reconstitution activity, comprising the method according to the present invention, wherein the detection of Miwi2/Piwi14^{Hi} c-kit^{Neg} spermatogonial stem cells (SSCs) is indicative for a testicular reconstitution activity, such as self-renewal or contribution to testicular homeostasis and regeneration.

Further preferred is the method according to the present invention, wherein said method is performed in the context of testicular cancer diagnosis.

Further preferred is the method according to the present invention, wherein said method is performed in the context of stem cell transplantation and/or stem cell transplantation assays.

Another aspect of the present invention then relates to a pharmaceutical composition according to the present invention for use in medicine.

Another aspect of the present invention then relates to a pharmaceutical composition according to the present invention for use in the prevention, improvement and/or treatment of testicular reconstitution, testicular regeneration, testicular self-renewal, testicular homeostasis, testicular stem cell transplantation and/or spermatogenesis.

Stem cell therapy provides new opportunities to replace and replenish damaged and dysfunctional testicular cells. Studies have demonstrated that transplantation of stem cells of testis origin into dysfunctional recipient testes can restore the capability to regenerate sperm. Preferred is the use in combination with high-dose chemotherapy (HDC).

Another aspect of the present invention then relates to an *in vitro* method for identifying a compound that improves testicular reconstitution activity, comprising a) contacting at least one candidate compound with a spermatogonial stem cell (SSC), and b) detecting the expression/activity of the markers Miwi2/Piwil4 and c-kit in said SSC, wherein an increase of the marker Miwi2/Piwil4 and a decrease of the marker c-kit in the presence of the candidate compound compared to the absence to the compound is indicative for a testicular reconstitution activity, such as self-renewal or contribution to testicular homeostasis and regeneration, for said at least one candidate compound.

Preferably, said compound is selected from the group consisting of a peptide library, a combinatory library, a cell extract, in particular a plant cell extract, a "small molecular drug", an antisense oligonucleotide, an siRNA, an mRNA and an antibody or fragment thereof specifically binding to Miwi2/Piwil4 and/or c-kit.

The method according to the present invention as described herein is thus suitable for the identification of compounds that can interact with the expression/activity of the marker Miwi2/Piwil4 and/or c-kit, and thus to identify, for example, inhibitors, competitors or modulators of the functions and/or expression of the markers.

The term "contacting" in the present invention means any interaction between the potentially binding substance(s) with the marker(s), whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip.

As a "function" of a marker, a biological function (like enzymatic function) and the changes thereof in the presence or absence of a compound is tested.

Also fragments of the polypeptides of the markers can be used in the assays of the invention, as long as they maintain the function to be tested and/or can be recognized in a detection assay, e.g. using specific antibodies and/or fragments thereof (e.g. scFV, Fab, etc.) as described herein.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, CI or Br, the introduction of lower alkyl groups, preferably having one to five carbon atoms like, for example, methyl, ethyl, n-propyl, iso propyl, n-butyl, iso butyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

If needed the steps of selecting the binding compound, modifying the binding compound, contacting the binding compound with a marker polypeptide and measuring the binding of the modified compounds to the protein can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate or modulate the activity of the marker polypeptide(s) as described herein.

Similar to the strategies for identifying compounds that interact with the markers, and/or the biological activity thereof, compounds can be identified that modulate the expression of the markers in a cell, e.g. the SSC. In preferred strategies, the expression of the marker(s) can be monitored using a genetic reporter-construct for the markers (in order to analyze the translation efficiency and/or stability of the marker polypeptide), for an example a fusion protein comprising a detectable fusion member (such as an enzymatic or fluorophoric group, or GFP), or the amount of mRNA as present in a cell can be measured, for example, by Northern blot. The expression can also be analyzed and monitored by using chip-analysis or rtPCR. Preferred compounds that modulate the expression of the markers in a cell are selected from specific antisense oligonucleotides, siRNAs, mRNAs or other preferably mutated nucleic acids encoding all or parts of the markers. Another preferred embodiment is the transfer of said genetic elements using gene therapy. Furthermore, encompassed are viral constructs for the introduction of said genetic elements into said cells. Alternatively, also the "naked" nucleic acid can be introduced into the cell(s), e.g. by using particle-mediated technologies. Respective methods are well described in the literature and known to the person of skill. Further preferred are screening tools according to the present invention as described herein, wherein said marker and/or the fragments thereof are labeled. Labels and methods for labeling are known to the person of skill, and can be enzymatic labels, dyes, fluorophores, and/or radioactive labels.

Preferred is a method according to the present invention as above, further comprising detecting the expression/activity of at least one marker selected from GFRα1^{Neg}, low side scatter (SSC^{lo}), and CD9, CD49f, Thy-1, CD29, and CD24 as described above.

Another aspect of the present invention then relates to a method for preventing, improving and/or treating testicular cancer, testicular reconstitution, testicular regeneration, testicular self-renewal, testicular homeostasis, testicular stem cell transplantation and/or spermatogenesis, comprising administering to said patient an effective amount of a pharmaceutical composition according to the invention as above. In general, the attending physician will base a treatment on the compound as identified, and optionally also on other individual patient data (clinical data, family history, DNA, etc.), and a treatment can also be performed based on the combination of these factors. This method of the present invention for example involves integrating individual diagnostic cancer data with patient clinical information and general healthcare statistics to enable, for example, the application of personalized medicine to the patient. Significant information about drug effectiveness, drug interactions, and other patient status conditions can be used, too. Preferred is a therapeutic method according to the present invention, wherein said mammal to be treated is a mouse, rat or human.

An "effective amount" is an amount of the compound(s) or the pharmaceutical composition as described herein that alleviates symptoms of the disease or condition as found for the patient, such as testicular reconstitution, testicular regeneration, testicular self-renewal, testicular homeostasis, testicular stem cell transplantation and/or spermatogenesis and/or testicular cancer. Alleviating is meant to include, e.g., preventing, treating, reducing the symptoms of, or curing the disease (e.g. testicular cancer) or condition (e.g. testicular reconstitution, testicular regeneration, testicular self-renewal, testicular homeostasis, testicular stem cell transplantation and/or spermatogenesis).

Another aspect of the present invention relates to a method or use as described herein, wherein the pharmaceutical composition further comprises additional pharmaceutically active ingredients for treating cancer, i.e. chemotherapeutics, e.g. in the context of HDC.

Another aspect of the present invention relates to a method or use as described herein, wherein said method is performed in the context of a stem cell transplantation and/or stem cell transplantation assays.

Another aspect of the present invention relates to a kit for detecting the mammalian spermatogonial stem cell according to the present invention, comprising materials for detecting the expression of the markers Miwi2/Piwil4 and c-kit, optionally together with materials for detecting the expression of the markers GFRα1, SSC^{lo}, CD9, CD49f, Thy-1, CD29, and CD24, and further optionally comprising additional compounds and instructions for performing the methods as described herein, such as materials for FACS, immunohistochemistry, and western blots, specific antibodies, as well as materials for immunohistochemical analyses. Additional materials include primers and probes for rtPCR and/or mRNA expression analyses.

Another aspect of the present invention relates to the use of the kit according to the present invention for detecting testicular reconstitution activity, self-renewal, a contribution to testicular homeostasis and regeneration and/or testicular cancer as described above.

The following figures, sequences, and examples merely serve to illustrate the invention and should not be construed to restrict the scope of the invention to the particular embodiments of the invention described in the examples. All references as cited herein are hereby incorporated in their entirety by reference.

In the Figures:

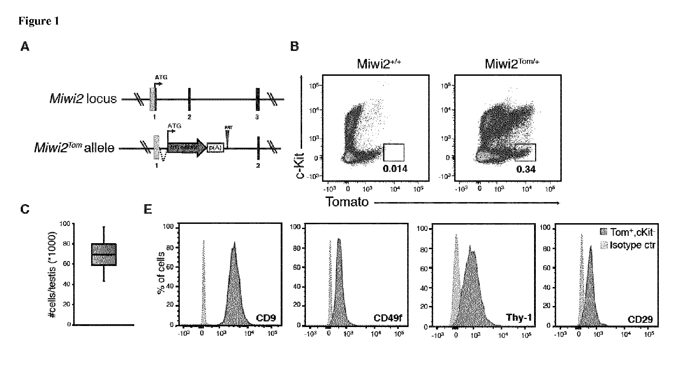

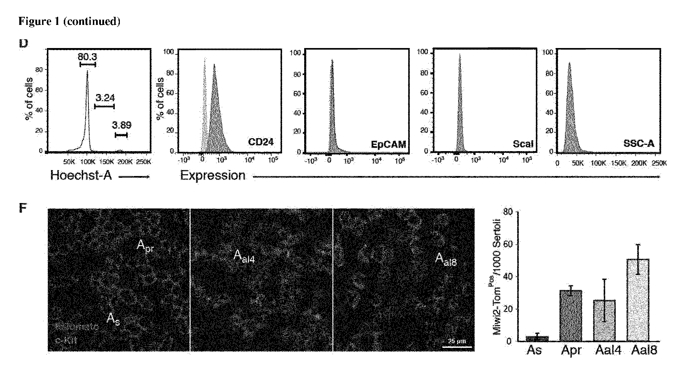

Figure 1 shows that Miwi2 Tomato expression defines a small population of undifferentiated spermatogonia. (A) Schematic over of the 5' region of the Miwi2 locus (upper panel) and the Miwi2 tdTomato transcriptional reporter allele (lower panel). (B) Representative FACS analysis of live CD45_{Neg} CD51_{Neg} gated cells is shown of testicular populations of wild type and Miwi_{tdTomato/+} mice. Numbers indicate the percentages of cells of the defined subpopulations. (C) Enumeration of testicular CD45_{Neg} CD51_{Neg} Miwi2-Tom_{Pos} c-Kit_{Neg} cells. Numbers represent cell per testis. (D) Cell cycle parameters of CD45_{Neg} CD51_{Neg} Miwi2-Tom_{Pos} c-Kit_{Neg} cells as determined by DNA content. (E) Cell surface expression by FACS of the indicated markers in CD45_{Neg} CD51_{Neg} Miwi2-Tom_{Pos} c-Kit_{Neg} are shown as well as isotype control staining. (F) Wholemount immuno-staining of Miwi_{tdTomato/+} seminiferous tubules with tdTomato and c-Kit antibodies. Aₛ and Aₚᵣ indicate A-single and A-paired undifferentiated spermatogonia, respectively. Enumeration of Miwi2-Tom_{Pos} Aₛ, Aₚᵣ, Aₐₗ₄ and Aₐₗ₈ undifferentiated spermatogonia normalized to 1000 sertoli cells.

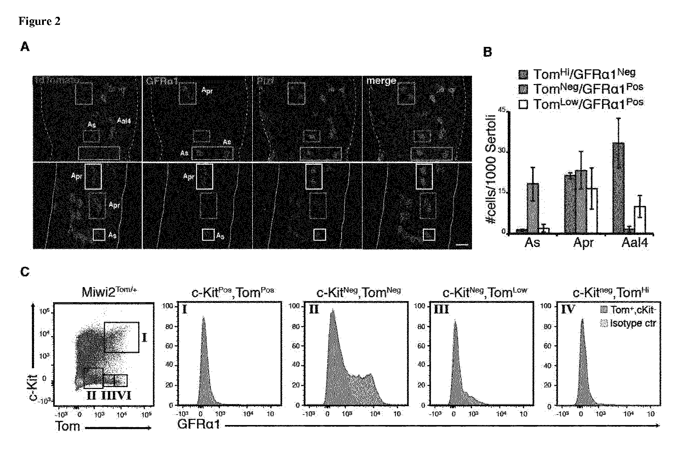

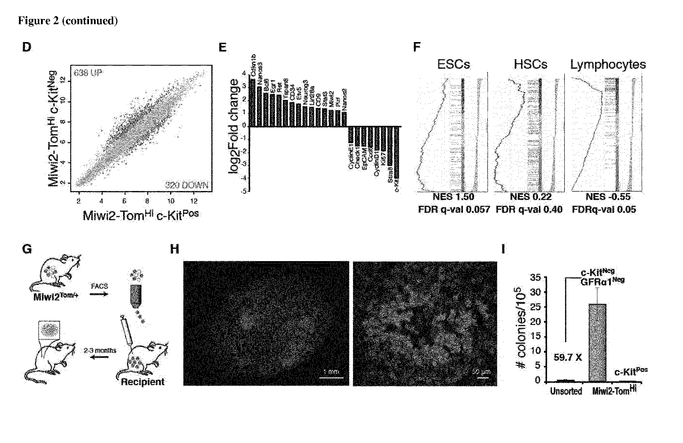

Figure 2 shows that Miwi2-TomHi c-KitNeg GFRα1Neg have spermatogonial stem cell activity. (A) Representative images of MiwitdTomato/seminiferous tubules stained with α- GFRα1 (Green), α-tdTomato (Red) and α-Plzf (Blue). Representative examples of Miwi2-TomHi GFRα1Neg (red box), Miwi2-TomNeg GFRα1Pos (green box) and Miwi2-TomLo GFRα1Pos (white box) populations are highlighted. (B) Enumeration of testicular populations defined in A. Numbers represents category normalized to 1000 sertoli cells. (C) Representative FACS analysis of GFRα expression on the indicated populations from gated CD45Neg CD51_{Neg} testicular MiwitdTomato/+ cells. (D) Scatter plot of gene expression from biological quadruplicates of CD45Neg CD51_{Neg} Miwi2-TomHI c-Kit_{Neg} and CD45Neg CD51_{Neg} Miwi2-TomHi c-KitPos populations. Genes highlighted in red show a fold change greater than equal to log2 1 and a significance of 0.05 or less. Number of genes up and down regulated in the respective populations are indicated. (E) Enrichment and depletion of select genes in CD45Neg CD51_{Neg} Miwi2-TomPos c-Kit_{Neg} population. (F) Gene set enrichment analysis (GSEA) comparing Miwi2-TomHi c-Kit_{Neg} gene expression to that of embryonic stem cells (ESCs), hematopoietic stem cells (HSCs) and lymphoid gene expression signatures. (G) Overview of transplantation experiment. (H) Representative image of reconstitution from CD45Neg CD51_{Neg} Miwi2-TomHi c-Kit_{Neg} transplanted cells isolated from *Miwi2Tom*/+; *Rosa26YFP*/+ mice. Colonies derived from the YFP-positive donor cell is shown. (I) Quantitation of colony formation for the indicated populations analyzed approximately 11 months after transplantation. Fold enrichment over nonsorted cell for the CD45Neg CD51_{Neg} Miwi2-TomHi c-Kit_{Neg} cells is shown.

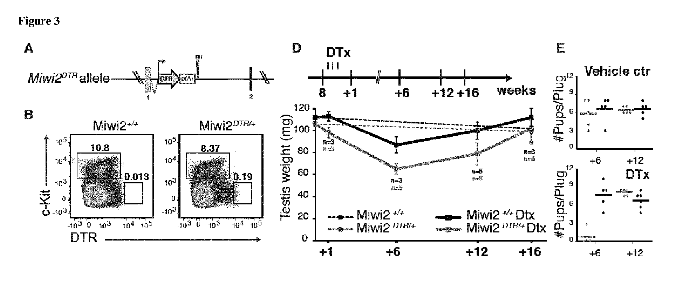

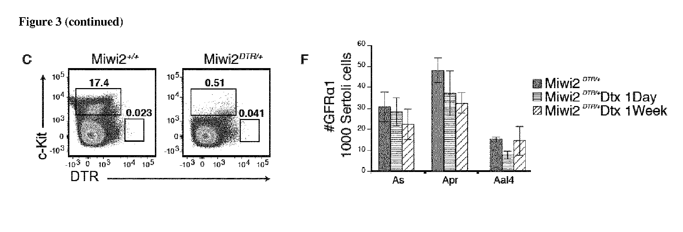

Figure 3 shows that Miwi2 expressing cells do not contribute to the long-term maintenance of spermatogenesis. (A) Schematic overview of the Miwi2_{DTR} allele. (B-C) Representative FACS analysis of live CD45Neg CD51_{Neg} gated cells is shown of testicular populations of wild type and Miwi_{DTR/+} mice stained with c-Kit and DTR antibodies (B) and after 3x DTx administrations analyzed 1 day after the last injection (C). Numbers indicate the percentages of cells of the defined subpopulations. (D) Experimental overview of DTx injections and time points analyzed (upper). Testicular weight of the indicated cohorts overtime is shown. n indicates number of mice analyzed per time point. (E) Fertility as represented by pups per plug of the indicated cohorts at 6 and 12 weeks post DTx or vehicle control injection. (F) Enumeration of the indicated GFRα1Pos populations in adult Miwi2_{DTR/+} mice that were untreated or treated with 3x DTx injections and analyzed 1 day and 1 week after the last injection. Numbers represents category normalized to 1000 sertoli cells.

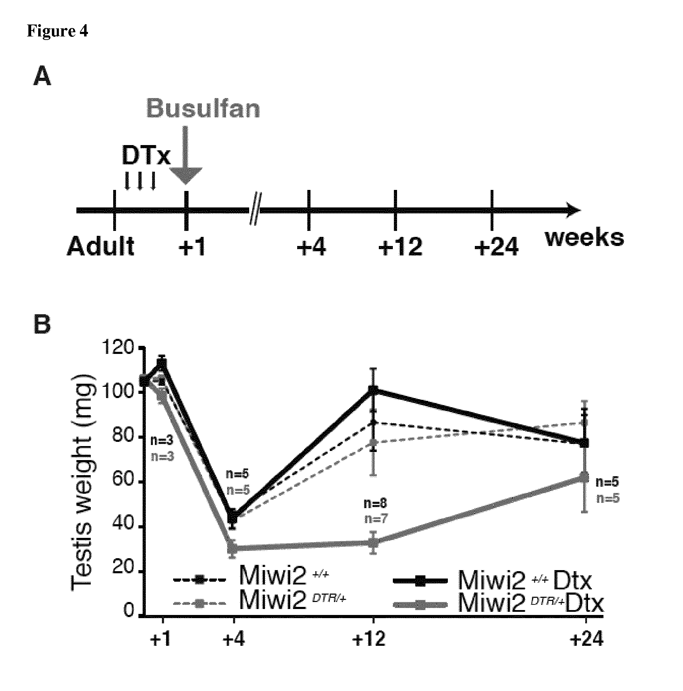

Figure 4 shows that Miwi2 expressing cells are crucial for the regenerative capacity of the testis after injury. (A) Experimental overview of DTx and busulfan injections as well as time points analyzed. (B) Testicular weight of the indicated cohorts overtime is shown. n indicates the number of mice analyzed per time point for the DTx injected mice.

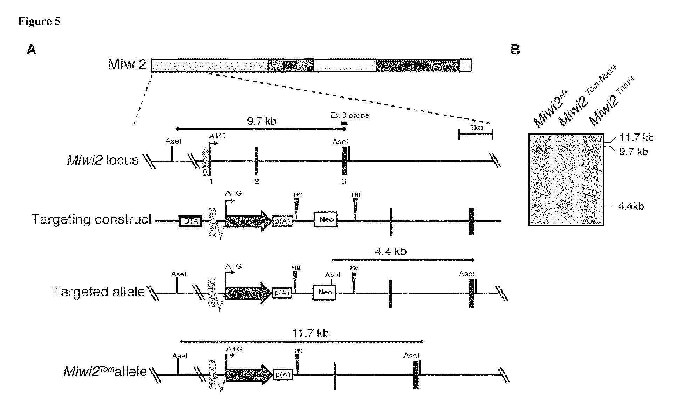

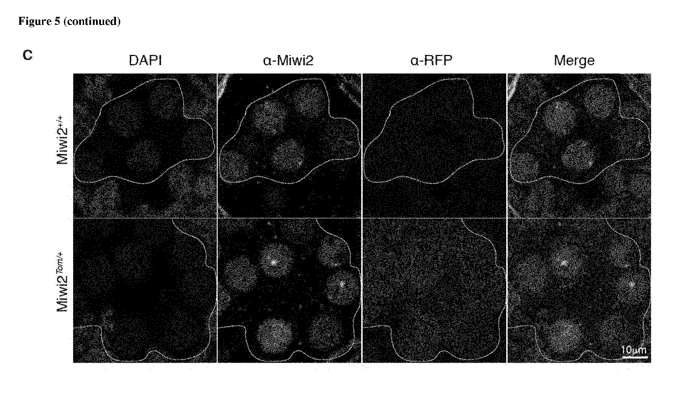

Figure 5 shows the generation of the *Miwi2_{tdTomato}* mouse allele. **(A)** Overview of a portion of the *Miwi2* locus that encodes 5' portion of the gene. The targeting vector used for introduction of *tdTomato* into the *Miwi2* locus and the schematic map of the targeted *Miwi2* gene before and after Flp mediated-recombination are shown. Triangles represent *frt* sites as indicated. Rectangles indicate the position of *Neomycin (Neo)* and *Diphtheria toxin A (DTA)* selection marker genes. The AseI restriction sites are indicated as well as the respective Southern fragments detected by the external 3'probe. **(B)** A Southern blot of tail derived AseI-digested DNA from wild-type, *Miwi2+*/*Tom-^{Neo}* and *Miwi2*+/Tom mice is shown with the 3' probe indicated in A. **(C)** Immunofluorescence using, DAPI, anti-Miwi2 and anti-RFP (recognizes tdTomato) antibodies on E16.5 fetal testis sections are shown.

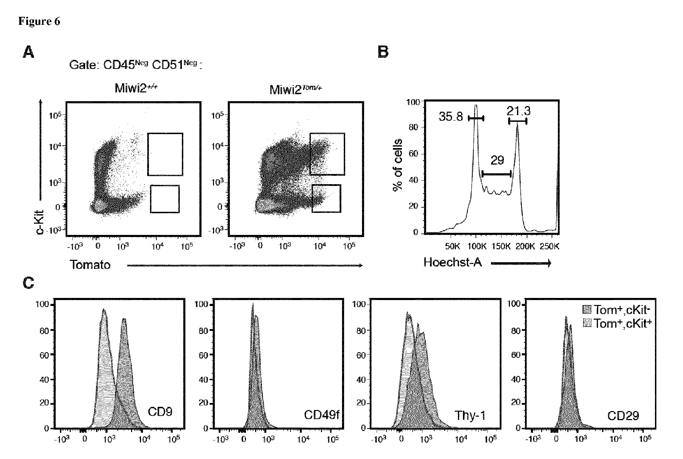

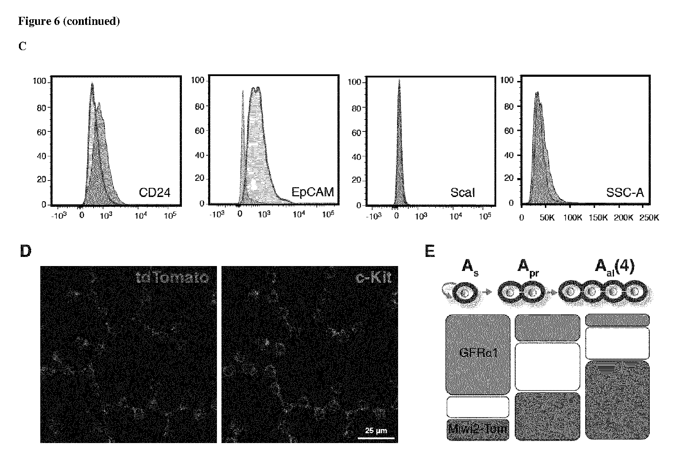

Figure 6 shows the characterization of the CD45_{Neg} CD51_{Neg} Miwi2-Tom_{Pos} c-KitPos testicular population. **(A)** Representative FACS analysis of live CD45_{Neg} CD51_{Neg} gated cells is shown of testicular populations of wild type and Miwi_{tdTomato/+} mice. Numbers indicate the percentages of cells of the defined subpopulations. **(B)** Cell cycle parameters of CD45_{Neg} CD51_{Neg} Miwi2-Tom_{Pos} c-KitPos cells as determined by DNA content. **(C)** Cell surface expression by FACS of the indicated markers in CD45_{Neg} CD51_{Neg} Miwi2-Tom_{Pos} c-Kit_{Neg} (grey histograms) and CD45_{Neg} CD51_{Neg} Miwi2-Tom_{Pos} c-KitPos (blue histograms) are shown. **(D)** Wholemount immuno-staining of Miwi_{tdTomatp/+} seminiferous tubules with tdTomato and c-Kit antibodies. Aₛ and Aₚᵣ indicate A-single and A-paired undifferentiated spermatogonia, respectively shown long chains of differentiating spermatogonia. **(E)** Summary of data shown in Figure 2B. Miwi2-Tom_{Hi} GFRα1_{Neg} (red box), Miwi2-Tom_{Neg} GFRα1_{Pos} (green box) and Miwi2-Tom_{Lo} GFRα1_{Pos} (white box) populations are depicted in Aₛ, Aₚᵣ and Aₐₗ₄ undifferentiated spermatogonia.

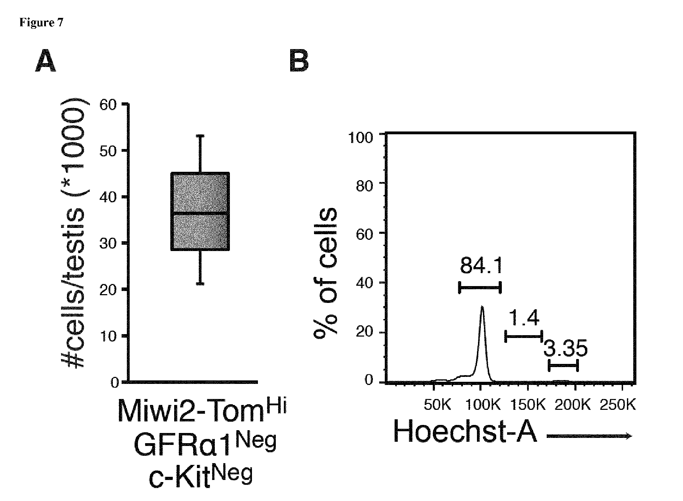

Figure 7 shows the numbers and cell cycle parameters of the Miwi2-TomHi c-Kit_{Neg} GFRα1_{Neg} population. **(A)** Enumeration of testicular CD45_{Neg} CD51_{Neg} Miwi2-Tom_{Hi} c-Kit_{Neg} GFRα1_{Neg} cells by FACS. Numbers represents cells per testis. **(B)** Cell cycle parameters of CD45_{Neg} CD51_{Neg} Miwi2-Tom_{Hi} c-Kit_{Neg} GFRα1_{Neg} cells as determined by DNA content.

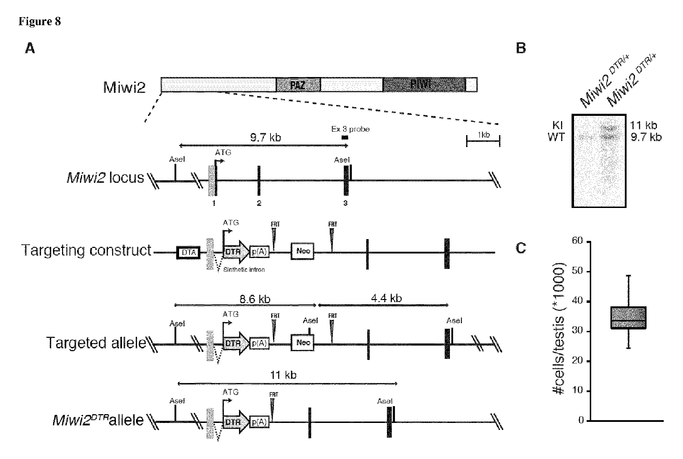

Figure 8 shows the generation and characterization of the Miwi2_{DTR} mouse allele. **(A)** Overview of a portion of the *Miwi2* locus that encodes 5' portion of the gene. The targeting vector used for introduction of *diphtheria toxin receptor (DTR)* into the *Miwi2* locus and the schematic map of the targeted *Miwi2* gene before and after Flp mediated recombination are shown. Triangles represent *frt* sites as indicated. Rectangles indicate the position of *Neomycin (Neo)* and *Diphtheria toxin A (DTA)* selection marker genes. The AseI restriction sites are indicated as well as the respective Southern fragments detected by the external 3'probe. **(B)** A Southern blot of tail derived AseI-digested DNA from wild-type, and *Miwi2*+/DTR mice is shown with the 3' probe indicated in A. **(C)** Enumeration of testicular CD45_{Neg} CD51_{Neg} Miwi2-DTR_{Pos} c-Kit_{Neg} cells by FACS. Numbers represents cells per testis.

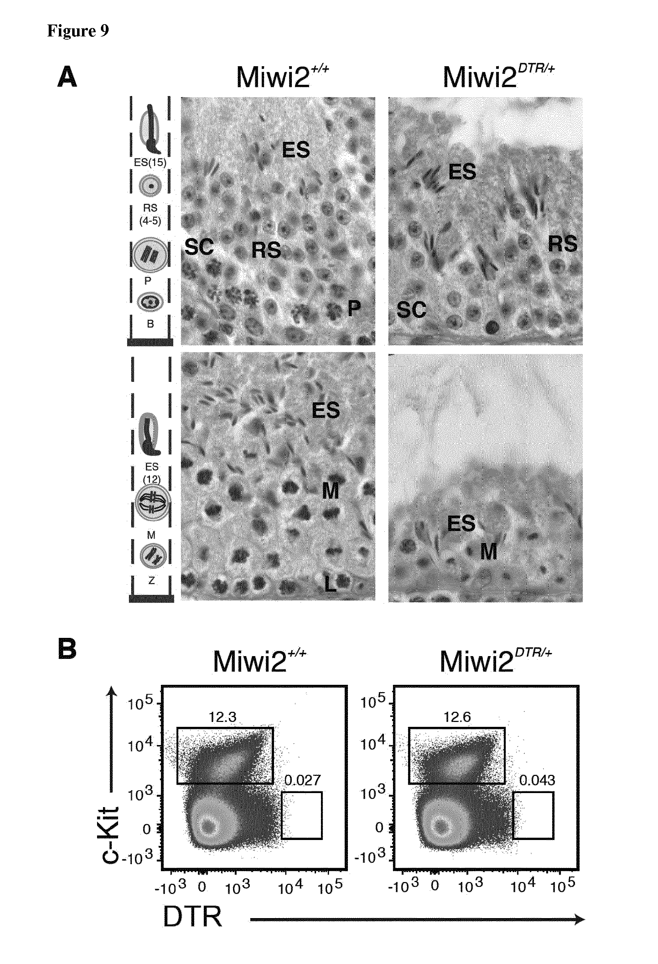

Figure 9 shows the effects of DTx administration on testis in Miwi2_{DTR/+} mice. **(A)** Histology of testis section stained with H&E in wild type and Miwi_{DTR/+} mice after 3x DTx administrations analyzed 1 day after the last injection. Stage 2-3 (upper panel) and 12 (lower panel) seminiferous tubules are shown. **(B)** Representative FACS analysis of live CD45_{Neg} CD51_{Neg} gated cells is shown of testicular populations of wild type and Miwi_{DTR/+} mice stained with c-Kit and DTR antibodies and after a single DTx administration and analyzed 1 day later. Numbers indicate the percentages of cells of the defined subpopulations.

### EXAMPLES

### Mouse Strains

The Miwi2_{tdTomato} and Miwi2_{DTR} were generated by standard gene targeting techniques in embryonic stem cells (ESCs) that were used to create fully derived ESC-mice.

### Cell preparation and FACS staining

Testes were isolated in DMEM enriched with 1mM of sodium pyruvate and 1.3 gr/ml of sodium lactate, carefully dealbuginated and rinsed twice before enzymatic digestion. To achieve the complete single-cell suspension the tubules were digested first with 0.5 mg/ml of type XI collagenase (Sigma c7657-500mg) at 32°C for 7 min at 700rpm shaking followed by 0.05% Trypsin digestion (Gibco 11500636) in the presence of 0.05 mg/ml of DNAse I (Sigma DN-25-100ug). After digestion, cells are filtered throughout 70 um cell strainer (Falcon 14-959-49A) and subsequently pelleted by centrifugation at 1000 rpm for 5 min. 5 to 7 million cells per sample were stained with the fluorophore-conjugated antibodies in PBS/FBS 3% sodium azid 0.01% for 30 min on ice. For the DTR and GFRα1 FACS staining cells were incubated with anti-HB EGF (DTR) biotinylated antibody (R&D System BAF259 clone 4G10) or α-GFRa1 antibody (Neuronics GT15004 Goat-IgG) diluted 1:50 in PBS 3%FBS 0.01% sodium azid and incubated on ice for 30 min with periodic mixing of the sample every 5 min. After washing in PBS-FBS cells were then incubated on ice for 30 min with either streptavidin pe-Cy7A (eBioscience 25-0441-82) or 647Alexafuor donkey antigoat antibody for DTR and GFRα1 staining, respectively.

### Antibodies used in this invention

FACS antibodies used were CD45 (eBioscience, 13-0451 clone A20), CD51 (Biolegend, 104104 clone RMV-7); CD90.2 (Biolegend clone 30-H12); CD29 (Biolegend 102224 clone hmbetal-1); CD9 (eBioscience 11-0091 clone KMC8); CD24 (Biolegend 101820 clone M1/69); CD34 (11-0341-81 clone RAM34); CD117 (eBioscience 25-1171 clone 2B8); Sca-1 (Biolegend 108119 D7); Cd49f (eBioscience 11-0495-80 clone GoH3) and EpCAM (eBioscience 11-5791-82 G8.8).

### Germ cell transplantation and reconstitution

Recipient animals were generated by single injection of busulfan at 40 mg/kg in 4-5 week old C57BL6n male mice (see below). Donor cells were isolated from *Miwi2*_{*Tom*/+}*; Rosa26* _{*YFP*/+} testis by FACS. Expression of YFP from the ubiquitously expressed Rosa26 locus was used to trace the reconstitution capacity of the donor cells. Germ cells were isolated from adult testes (8-10 weeks) of *Miwi2*_{*Tom*/+}*; Rosa26* _{*YFP*/+} mice and FACS sorted in PBS 10%FCS as described above without sodium azide in the solution. 10₄ Miwi2-Tom_{HI}, c-kit_{Pos} or Miwi2-Tom_{HI}, c-kit_{Neg} cells or 10⁶ unsorted cells were resuspended in a volume of 10ul in fresh DMEM/0.05% trypan blue and loaded into a glass pipet. The glass pipet with a 1-mm outside diameter and a 40um beveled tip was secured in a micropipette-holder (WPI Instruments (Waltham, MA)) and used for delivery of the various cell populations. Surgery was performed under isoflurane-oxygen vapor anesthesia. The capillary needle was gently inserted into the rete-testis, through the efferent duct of the recipient animal and approximately 10 ul of the cell solution was transplanted. The injection filled 75-85% of the tubules in each recipient testis. 50µg anti-CD4 antibody (GK1.5) was intraperitoneally injected to recipient mice on days 0, 2, and 4 after transplantation to prevent potential immune rejection to the allogeneic donor cells. To count colonies, recipient mouse testes were recovered approximately 11 weeks after transplantation and analyzed by observing the number of YFP fluorescent colonies formed. A cluster of germ cells was defined as a colony when it occupied the entire circumference of the tubule and was at least 0.1 mm long.

### Whole mount Immunofluorescence

Tubules are dealbugined with 0.5mg/ml collagenase as for FACS preparation (above) and subsequently fixed in 4% paraformaldehyde for 8-12 hours at 4°C. After fixation tubules are quenched in 1M glycine for 30 min at room temperature and then blocked/permeabilized in 0.5% Triton X-100, donkey serum 5% and BSA 0.5%.

Primary antibodies are incubated in PBS 0.5% BSA over night at room temperature. After washes in PBS, the tubules were incubated with either Alexa-488, 647 or 546 conjugated secondary antibodies in PBS 0.5%BSA Triton X 0.1%. The tubules were then counterstained with DAPI (5 ug/ml) and mounted in prolong gold on glass slides. Antibodies used were α-GFRα1 (Neuronics GT15004 Goat-IgG), α-Plzf (2A9, Calbiochem, mouse monoclonal), α-Plzf (H-300, Santa Cruz, rabbit polyclonal) and c-kit (R&D, goat polyclonal).

### Lineage ablation

DTx (Sigma) was dissolved in water to make high concentration aliquots (1mg/ml) that were kept as stocks at -80°C. For injections the concentrated DTx stock was diluted in PBS for the working concentration (5µg/ml). Miwi2 _{DTR/+} mice were injected intraperitoneally 3 times every second day (unless specified otherwise) with DTx at a concentration of 25ng DTx per gram of mouse body weight.

### Busulfan treatment of mice.

Busulfan powder was dissolved in dimethyl sulfoxide (DMSO) prewarmed to 41°C-43°C. After the busulfan was dissolved, an equal volume of distilled water (41°C-43°C) is added to make the working solution. For elimination of germ line to generate recipient mice for the transplantation assays, a single busulfan injection was administered at a dose of 40 mg/kg of mouse body weight. For the regeneration experiment the Miwi2 _{+/+} or Miwi2 _{DTR/+} mice were injected with busulfan at 20 mg/kg of mouse body weight the day after the last DTx injection. Analysis of regeneration was performed at the time points described in the text and figure legends.

Among the loci required for the maintenance of the male germ line, the gene encoding the Piwi protein Miwi2 caught the inventors' attention due to the slow progressive loss of germ cells that is observed in Miwi2_{-/-} mice. In addition Miwi2's reported expression domain is restricted to gonadocytes that are undergoing *de novo* DNA methylation rather than a population of adult spermaotogonia. The inventors therefore reasoned that Miwi2 could be additionally expressed in a tiny population of adult spermatogonia with SSC activity that has been overlooked by virtue of its rarity. To test this hypothesis the inventors generated a transcriptional reporter *(Miwi2_{Tom})* allele whereby the gene encoding the fluorescent tdTomato protein was knocked into the first coding exon of *Miwi2* tdTomato faithfully recapitulating the expression of Miwi2 in Miwi2_{+/Tom} reprogramming gonadocytes (Fig. 1A and Fig. 5). Next the inventors examined by FACS Miwi2-tdTomato expression in the testis gating out somatic populations with CD45 and CD51, the inventors observed a tiny tdTomato positive c-kit negative (Miwi2-Tom_{Pos} c-Kit_{Neg}) population (Fig. 1B) and a larger positive (Miwi2-Tom_{Pos} c-Kit_{Pos}) that constitute proliferating EpCAM-positive differentiating spermatogonia (Fig. 6).

Sorting of these respective populations revealed Miwi2 transcript in the Miwi2-Tem_{Pos} c-Kit_{Neg}, but not in the Miwi2-TomPos c-KitPos populations (data not shown). The inventors therefore concluded that the tdTomato expression in Miwi2-TomPos c-Kit_{Pos} population reflects the extended life of the tdTomato protein rather the active expression of the *Miwi2* gene *per se,* similar as to what is observed in the descendants of Lgr5-GFP_{HI} intestinal stem cells. c-Kit negativity is a hallmark of SSC populations, the inventors therefore focused the inventors' attention on the Miwi2-TomPos c-Kit_{Neg} population that represents approximately 70 thousand cells per testis and are mostly quiescent (Fig 1C and D). The inventors next sought to define the surface phenotype of Miwi2-TomPos c-Kit_{Neg} cells, this population uniformly expresses all the markers (CD9, CD49f, Thy-1, CD29, CD24) as well as being low side scatter (SSCₗₒ) (Fig. 1E), all attributes that have been shown to enrich for SSC activity in transplantation assays. Furthermore this population is not only negative for c-Kit but also Sca1 (Fig. 1E) whose expression has been shown to deplete for SSC potential. The Miwi2-Tom_{Pos} c-Kit_{Neg} population constitutes a tiny population Aₛ as well as a greater quantity of Apr and Aₐₗ undifferentiated spermatogonia (Fig. 1F). In summary the Miwi2_{Tom} allele can used to define c-kit negative population of mostly quiescent A type undifferentiated spermatogonia with a very defined surface phenotype associated with stem cell activity.

The inventors next sought to relate the inventors' Miwi2-TomPos c-Kit_{Neg} population to GFRα1-expressing SSCs as well as Plzf-expression that encompasses a larger population of c-kit negative spermatogonial precursor cells. All Miwi2-TomPos c-Kit_{Neg} were Plzf positive (Fig. 2A). Analysis of GFRα1 and Tomato expression in As, Apr and Aₐₗ₄ revealed three distinct populations of Miwi2-Tomato and GFRα1 expressing cells, the first group were positive for Miwi2-tdTomato only (Miwi2-Tom_{Hi} GFRα1_{Neg}), the second class was solely Gfra1 positive (Miwi2-Tom_{Neg} GFRα1_{Pos}) and the third subset expressed low amounts of Miwi2-tdTomato but were GFRα1positive (Miwi2-Tom_{Lo} GFRα1_{Pos}) (Fig. 2A). The majority of As were Miwi2-Tom_{Neg} GFRα1Pos with the other two populations present but contributing minimally (Fig. 2B). An expansion of the Miwi2-TomHi GFRα1Neg and Miwi2-Tom_{Lo} GFRα1_{Pos} population was observed with roughly all three groups contributing equally to Apr spermatogonia (Fig. 2B). At the Aₐₗ₄ stage the majority of cells had lost GFRα1 expression and the Miwi2-TomHi GFRα1Neg dominated this population (Fig. 2B). By Aₐₗ₈, all cells were Miwi2-TomHi GFRα1_{Neg}. These data suggested that high levels of Miwi2-Tom expression can be used to define a population of GFRα1-negative undifferentiated spermatogonia.

Indeed, FACS analysis of Miwi2-TomHi c-Kit_{Neg} cells revealed their GFRα1 negativity, with increasing GFRα1 expression in cells that were Miwi2 low to dull c-Kit_{Neg} cells (Fig. 2C). This Miwi2-TomHi c-Kit_{Neg} GFRα1Neg population constituted roughly 35 thousand cells and again was mostly quiescent (Fig. 7). Comparative gene expression analysis between Miwi2-TomHi c-Kit_{Neg} GFRα1Neg population and their Miwi2-TomHi c-KitPos descendants revealed many differences in the respective transcriptomes (Fig. 2D). Enrichment for many genes that have been associated with the SSC phenotype or function was found in the Miwi2-TomHi c-Kit_{Neg} GFRα1Neg population (Fig. 2E), conversely depletion for differentiating spermatogonia expressed genes were observed in the same population (Fig. 2E). Gene set enrichment analysis (GSEA) revealed that the gene expression in the Miwi2-TomHi c-Kit_{Neg} GFRα1Neg bore similarity to ESCs, less so to HSCs but was very distinct to lymphocytes (Fig. 2F). Collectively, all the data point to a potential stem cell function for Miwi2-TomHi c-Kit_{Neg} GFRα1Neg population. Indeed the Miwi2-TomHi c-Kit_{Neg} GFRα1Neg population but not Miwi2-TomHi c-Kit_{Pos} cells contained robust reconstitution activity in transplantation assays (Fig. 2G and H).

To determine the contribution of the Miwi2-TomHi c-Kit_{Neg} GFRα1Neg SSC containing population to the steady state or the long-term maintenance of spermatogenesis the inventors opted for a lineage ablation strategy. To this end, the gene encoding the diphtheria toxin receptor (DTR) was placed into the Miwi2 locus (*Miwi2_{DTR}* allele) (Fig. 3A and 8), thus enabling diphtheria toxin (DTx)-mediated linage ablation experiments. Unfortunately, the DTR antibody did not work for wholemount immunostaining, so that the inventors could not resolve the DTR-expressing cells at the cellular Aₛ-Aₐₗ resolution. However, DTR expression was determined by FACS and was restricted to a tiny population of CD45Neg CD51_{Neg} c-Kit_{Neg} (Miwi2-DTR_{Pos} c-Kit_{Neg}) cells but not in any c-KitPos population within Miwi2_{+/DTR} testis (Fig. 3B).

Importantly, the overall percentages and numbers of Miwi2-DTR_{Pos} c-Kit_{Neg} and Miwi2-TomHi c-Kit_{Neg} were roughly equivalent (Fig 3B and 8). The inventors administered three DTx doses spaced two days apart and analyzed one day after the last injection, here the Miwi2-DTR_{Pos} c-Kit_{Neg} population was reduced close to background levels (Fig 3C). This acute loss of Miwi2-DTR_{Pos} c-Kit_{Neg} population had no impact on Gfra1-expressing SSC populations (Fig. 3D), however strikingly most differentiating spermatogonia (CD45_{Neg} CD51_{Neg} c-Kit_{Pos}) were absent (Fig 3F). The inventors interpreted this loss of c-KitPos cells as the failure to replenish the differentiating spermatogonia population upon their differentiation into meiocytes, indeed histological analysis supported this (Fig. S5A). Indeed a single administration of DTx and analysis one day after the injection revealed the Miwi2-DTR_{Pos} c-Kit_{Neg} population was reduced but without affecting other testicular inclusive of the c-Kit_{Pos} populations (Fig S5B), thus the DTx is not directly targeting the c-Kit population. The inventors next followed the consequence of Miwi2-DTR_{Pos} c-Kit_{Neg} depletion on spermatogenesis over time, a wave of spermatogenesis was lost in the Miwi2_{DTR/+} DTx treated mice with testicular weight being reduced by 45% 6 weeks after injection but recovering fully by 16 weeks (Fig. 3D). Concomitantly fertility was lost at 6 weeks and regained at 12 weeks post DTx injection in the Miwi2_{DTR/+} mice (Fig. 3E). Thus the Miwi2-DTR_{Pos} c-Kit_{Neg} population behaves as transit amplifying population in this assay and the cells within this population with SSC activity are not broadly required for the homeostasis of the testis.

Miwi2 is member of the Piwi sub-clade of the Argonaute family, in planarians the Piwi proteins Smedwi2 and Smedwi2 are expressed in neoblasts and essential for the remarkable regenerative capacity of this organism. The inventors therefore sought to understand if the Miwi2-DTR_{Pos} c-Kit_{Neg} population contributes to the regenerative capacity of the testis upon injury. Busulfan is a DNA alkylating agent that is particularly toxic to spermatogonia, at intermediate doses this chemical can be used to damage testis and induce regeneration. The inventors subjected a cohort of Miwi_{+/+} and Miwi_{+/DTR} to either vehicle control or 3x DTx injections immediately followed by treatment of the mice with an intermediate dose of busulfan (Fig. 4A) The regeneration of vehicle control injected mice was identical between the control and experimental genotypes (Fig 4B).

However within the cohort where DTx was administered the *Miwi2*+/DTR mice failed to recover with the same kinetics as control mice. At 12 weeks post busulfan injection the wild type mice had reached maximal recovery whereas the *Miwi2*+/DTR mice showed little or no recovery at this time point (Fig. 4B). Only at 24 weeks post injury did the DTx-injected *Miwi2*+/DTR mice mount a significant recovery (Fig. 4B).

Collectively these data demonstrate the Miwi2-DTR_{Pos} c-Kit_{Neg} cells contain an SSC population that is required for the efficient regenerative capacity of the testis after injury.

## Claims

1. An isolated mammalian spermatogonial stem cell (SSC) that is Miwi2/Piwi14^{Hi} c-kit^{Neg}.

2. The SSC according to claim 1, which is further selected from GFRα1^{Neg}, low side scatter (SSC^{lo}), and CD9^{Pos}, CD49f^{Pos}, Thy-1 ^{Pos}, CD29^{Pos}, and CD24^{Pos}.

3. A pharmaceutical composition, comprising an SSC according to claim 1 or 2, and a pharmaceutically acceptable carrier.

4. An *in vitro* method for detecting a mammalian spermatogonial stem cell population, comprising detecting the expression of the markers Miwi2/Piwil4 and c-kit, optionally together with GFRα1, SSC^{lo}, CD9, CD49f, Thy-1, CD29, and CD24.

5. An *in vitro* method for detecting testicular reconstitution activity, comprising the method according to claim 4, wherein the detection of Miwi2/Piwi14^{Hi} c-kit^{Neg} spermatogonial stem cells (SSCs) is indicative for a testicular reconstitution activity, such as self-renewal or contribution to testicular regeneration.

6. The method according to claim 4 or 5, wherein said method is performed in the context of testicular cancer diagnosis.

7. The method according to claim 4 or 5, wherein said method is performed in the context of stem cell transplantation and/or stem cell transplantation assays.

8. A pharmaceutical composition according to claim 3 for use in medicine.

9. A pharmaceutical composition according to claim 3 for use in the prevention, improvement and/or treatment of testicular reconstitution, testicular regeneration, testicular self-renewal, testicular stem cell transplantation and/or spermatogenesis.

10. An *in vitro* method for identifying a compound that improves testicular reconstitution activity, comprising
a) contacting at least one candidate compound with a spermatogonial stem cell (SSC), and
b) detecting the expression/activity of the markers Miwi2/Piwil4 and c-kit in said SSC,
wherein an increase of the marker Miwi2/Piwil4 and a decrease of the marker c-kit in the presence of the candidate compound compared to the absence to the compound is indicative for a testicular reconstitution activity, such as self-renewal or contribution to testicular regeneration, for said at least one candidate compound.

11. The method according to claim 10, further comprising detecting the expression/activity of at least one marker selected from GFRα1^{Neg}, low side scatter (SSC^{lo}), and CD9^{Pos}, CD49f^{Pos}, Thy-1 ^{Pos}, CD29^{Pos}, and CD24^{Pos}.

12. A method for preventing, improving and/or treating of testicular reconstitution, testicular regeneration, testicular self-renewal, testicular homeostasis, testicular stem cell transplantation and/or spermatogenesis, comprising providing the pharmaceutical composition according to claim 3 to a patient in need thereof.

13. The method according to claim 12, wherein said method is performed in the context of a stem cell transplantation and/or stem cell transplantation assays.

14. A kit for detecting the mammalian spermatogonial stem cell according to claim 1 or 2, comprising materials for detecting the expression of the markers Miwi2/Piwil4 and c-kit, optionally together with materials for detecting the expression of the markers GFRα1, SSC^{lo}, CD9, CD49f, Thy-1, CD29, and CD24.

15. Use of the kit according to claim 14 for detecting testicular reconstitution activity, self-renewal, contribution to testicular regeneration or testicular cancer.
